(19) Europäisches Patentamt
European Patent Office
Office européen des brevets

(11) EP 4 285 856 A1

(12) EUROPEAN PATENT APPLICATION
published in accordance with Art. 153(4) EPC

(43) Date of publication:
06.12.2023  Bulletin 2023/49

(51) International Patent Classification (IPC):
A61B 34/30 (2016.01)

(21) Application number: 21922107.4

(86) International application number:
PCT/CN2021/093793

(22) Date of filing: 14.05.2021

(87) International publication number:
WO 2022/160509 (04.08.2022 Gazette 2022/31)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority:  27.01.2021  CN 202110107832

(71) Applicant: Harbin Intelligent Surgery Equipment
Co., Ltd.
Harbin, Heilongjiang 150000 (CN)

(72) Inventors:
• WANG, Wei
Harbin, Heilongjiang 150000 (CN)
• WEN, Yang
Harbin, Heilongjiang 150000 (CN)

(74) Representative: Zaboliene, Reda
Metida
Business center Vertas
Gyneju str. 16
01109 Vilnius (LT)

(54) COLLISION DETECTION METHOD AND APPARATUS FOR LAPAROSCOPIC MINIMALLY INVASIVE SURGICAL ROBOT

(57) The present disclosure discloses a collision detection method of a laparoscopic minimally invasive surgery robot, comprising: establishing an envelope model on a link, performing kinematic modeling of the envelope model, acquiring an electric motor code disc value and obtaining a real-time position of the envelope model according to the electric motor code disc value, obtaining a link distance between different surgical arms according to the real-time position of the envelope model, judging whether collision occurs between different surgical arms according to the link distance, and giving pre-warning prompt information when collision occurs between different surgical arms. According to the present disclosure, the real-time spatial position of the surgical arm can be obtained on the basis of the electric motor code disc value of the surgical arm, the reliance on detection apparatuses such as sensors can be reduced, integration problems during the use of multiple sensors can be eliminated and costs are saved. At the same time, through the active detection of the link distance during the operation of the surgical arms, the collision between the surgical arms and the interference with external equipment can be effectively pre-warned, which is conducive to the operation of laparoscopic minimally invasive surgery.

Fig.1

## Description

### Technical Field

The present disclosure relates to the technical field of collision detection, and more particularly, to a collision detection method and apparatus of laparoscopic minimally invasive surgery robot.

### Background Art

For the laparoscopic minimally invasive surgery robot, due to the limitation of the surgical environment and the space in human body, the positions of the channels for establishing micro-instruments on the body surface to enter and exit the body are less apart from each other, and the surgical environment is affected by more instruments and equipment for the surgical arm moving outside the body. Collisions between the surgical arms and interference of the surgical arms with other equipment can cause problems such as field of view sway, micro-instrument tip jitter, etc. which can greatly affect the minimally invasive surgical operation. There are two main treatment methods, wherein the first method is passive prevention by preoperative planning. According to the target surgical area and combined with the characteristics of surgical robot, the position of minimally invasive surgical incision is planned, the initial shape of mechanical arm is determined and enough space for external motion of surgical mechanical arm is reserved, so as to minimize the probability of interference between arm and arm, or between arm and other surgical equipment during surgery. The second method is to actively detect the distance between surgical arms by integrating corresponding detection sensors, so as to achieve the purpose of interference prediction.

However, with the first method, collision detection and prejudgment cannot be actively performed, while the second method relies too much on sensors and is difficult to integrate and expensive to use.

### Summary of the Invention

The problem to be solved by the present disclosure is how to design a collision detection method and apparatus of laparoscopic minimally invasive surgery robot with low cost and capable of actively performing collision detection prejudgement on a surgical arm.

In order to solve the problems, the present disclosure provides a collision detection method of a laparoscopic minimally invasive surgery robot, comprising:

> establishing an envelope model on a link,
> performing kinematic modeling of the envelope model,
> acquiring an electric motor code disc value and obtaining a real-time position of the envelope model according to the electric motor code disc value, obtaining a link distance between different surgical arms according to the real-time position of the envelope model,
> judging whether collision occurs between different surgical arms according to the link distance, and providing pre-warning prompt information when collision occurs between different surgical arms.

Optionally, a manner of establishing the envelope model comprises:
determining a position of a characteristic point on the link, and establishing the envelope model according to the characteristic point, a preset radius of the envelope model and a preset length of the envelope model.

Optionally, a shortest distance from any point on an axial segment of the envelope model to a housing of the envelope model is equal.

Optionally, a manner of obtaining a real-time position of the envelope model comprises: obtaining the real-time position of the characteristic point by performing a kinematic solution according to the electric motor code disc value, and obtaining the real-time position of the envelope model by calculating according to the real-time position of the characteristic point and the radius of the envelope model.

Optionally, a manner of obtaining a link distance between different surgical arms comprises: obtaining a shortest distance between two different axial segments according to a real-time position of the envelope model, and obtaining the link distance according to the shortest distance.

Optionally, a manner of obtaining a shortest distance between two different axial segments comprises:

> determining a common perpendicular line of axes of two different envelope models,
> judging whether intersection points of the common perpendicular line with axes of two different envelope models completely fall on two different axial segments,
> determining the shortest distance according to the following formula when the intersection points of the common perpendicular line with the axes of two different envelope models completely fall on two different axial segments:

$$k_1 = \frac{(P_{BR} - P_{BL})^2 \cdot (P_{AR} - P_{AL}) \cdot (P_{AL} - P_{BL}) - (P_{AR} - P_{AL}) \cdot (P_{BR} - P_{BL})^2 \cdot (P_{AL} - P_{BL})}{(P_{AR} - P_{AL})^2 \cdot (P_{BR} - P_{BL})^2 - (P_{AR} - P_{AL})^2 \cdot (P_{BR} - P_{BL})^2}$$

$$k_2 = \frac{(P_{AR} - P_{AL})^2 \cdot (P_{BR} - P_{BL}) \cdot (P_{AL} - P_{BL}) - (P_{BR} - P_{BL}) \cdot (P_{BR} - P_{BL})^2 \cdot (P_{AL} - P_{BL})}{(P_{AR} - P_{AL})^2 \cdot (P_{BR} - P_{BL})^2 - (P_{AR} - P_{AL})^2 \cdot (P_{BR} - P_{BL})^2}$$

$$P_{VA} = P_{AL} + k_1(P_{AR} - P_{AL})$$

$$P_{VB} = P_{BL} + k_2(P_{BR} - P_{BL})$$

wherein $P_{AL}$ and $P_{AR}$ are respectively two different characteristic points of one envelope model, while $P_{BL}$ and $P_{BR}$ are respectively two different characteristic points of the other envelope model, $P_{VA}$ being the intersection point of the common perpendicular line with the axis of one envelope model, $P_{VB}$ being the intersection point of the common perpendicular line with the axis of the other envelope model, and $D_P$ being the shortest distance,
determining the shortest distance according to the following formula when the intersection point of the common perpendicular line with the axes of two different envelope models fail to completely fall on two different axial segments:

$$D_{AL}^B = \begin{cases} \|P_{AL} - P_{BL}\|, k < 0 \\ \|P_{AL} - kP_{BR} - (1-k)P_{BL}\|, 0 \le k \le 1, k = \dfrac{(P_{BR} - P_{BL}) \cdot (P_{AL} - P_{BL})}{(P_{BR} - P_{BL}) \cdot (P_{BR} - P_{BL})} \\ \|P_{AL} - P_{BR}\|, k > 1 \end{cases}$$

$$D_{AR}^B = \begin{cases} \|P_{AR} - P_{BL}\|, k < 0 \\ \|P_{AR} - kP_{BR} - (1-k)P_{BL}\|, 0 \le k \le 1, k = \dfrac{(P_{BR} - P_{BL}) \cdot (P_{AR} - P_{BL})}{(P_{BR} - P_{BL}) \cdot (P_{BR} - P_{BL})} \\ \|P_{AR} - P_{BR}\|, k > 1 \end{cases}$$

$$D_{BL}^A = \begin{cases} \|P_{BL} - P_{AL}\|, k < 0 \\ \|P_{BL} - kP_{AR} - (1-k)P_{AL}\|, 0 \le k \le 1, k = \dfrac{(P_{AR} - P_{AL}) \cdot (P_{BL} - P_{AL})}{(P_{AR} - P_{AL}) \cdot (P_{AR} - P_{AL})} \\ \|P_{BL} - P_{AR}\|, k > 1 \end{cases}$$

$$D_{BR}^A = \begin{cases} \|P_{BR} - P_{AL}\|, k < 0 \\ \|P_{BR} - kP_{AR} - (1-k)P_{AL}\|, 0 \le k \le 1, k = \dfrac{(P_{AL} - P_{BL}) \cdot (P_{BR} - P_{AL})}{(P_{AR} - P_{AL}) \cdot (P_{AR} - P_{AL})} \\ \|P_{BR} - P_{AR}\|, k > 1 \end{cases}$$

$$D_P = \min(D_{AR}^B, D_{AL}^B, D_{BL}^A, D_{BR}^A)$$

wherein $DB_{AL}$ and $DB_{AR}$ are respectively the shortest distances of two different characteristic points $P_{AL}$ and $P_{AR}$ to one axial segment, and $DA_{BL}$ and $DA_{BR}$ are respectively the shortest distances of two different characteristic points $P_{BL}$ and $P_{BR}$ to the other axial segment.

Optionally, a manner of obtaining the link distance according to the shortest distance comprises:

$$D_V = D_P - r_{SA} - r_{SB}$$

wherein $r_{SA}$ is a radius of one envelope model, $r_{SB}$ is a radius of the other envelope model, and Dv is the link distance.

Optionally, the collision detection method of a laparoscopic minimally invasive surgery robot further comprises: defining a working interval of the surgical arm with a laser marking apparatus to define a range of motion of the surgical arm.

Optionally, a manner of judging whether collision occurs between different surgical arms according to the link distance comprises: comparing the link distance with a preset threshold value, and when the link distance is less than or equal to the threshold value, judging that collision occurs between different surgical arms, while when the link distance is greater than the threshold value, judging that no collision occurs between different surgical arms.

The present disclosure also discloses a collision detection apparatus based on a laparoscopic minimally invasive surgery robot, comprising:

an envelope module for establishing an envelope model on a link,

a modeling module for performing kinematic modeling of the envelope model so as to calculate a real-time spatial position of the envelope model,

a calculation module for acquiring an electric motor code disc value and obtaining a real-time position of the envelope model according to the electric motor code disc value and the established kinematic model, and for obtaining a link distance between different surgical arms according to the real-time position of the envelope model,

a pre-warning module for judging whether collision occurs between different surgical arms according to the link distance, and providing pre-warning prompt information when collision occurs between different surgical arms.

Advantageous Effects of the Invention: the real-time spatial position of the surgical arm can be obtained on the basis of the electric motor code disc value of the surgical arm. Compared with using external sensors such as distance measurement to detect the real-time spatial position of the surgical arm, the dependence on detection apparatuses such as sensors is reduced, the integration problem during the use of multiple sensors is eliminated and the cost is saved. At the same time, through the active detection of the link distance during the operation of the surgical arms, the collision between the surgical arms and the interference with the external equipment can be effectively warned, avoiding the intraoperative safety problems caused by the collision and facilitating the operation of laparoscopic minimally invasive surgery.

**Brief Description of the Drawings**

Fig. 1 is a flowchart I of a collision detection method of a laparoscopic minimally invasive surgery robot according to an embodiment of the present disclosure;

Fig. 2 is a flowchart II of a collision detection method of a laparoscopic minimally invasive surgery robot according to an embodiment of the present disclosure;

Fig. 3 is a schematic view showing the structure of an envelope model according to an embodiment of the present disclosure;

Fig. 4 is a schematic view showing an application of a collision detection model of a laparoscopic minimally invasive surgery robot according to an embodiment of the present disclosure.

**Detailed Description of the Invention**

To make the objects, features and advantages of the present disclosure more apparent, a detailed description of specific embodiments of the present disclosure will be made with reference to the accompanying drawings.

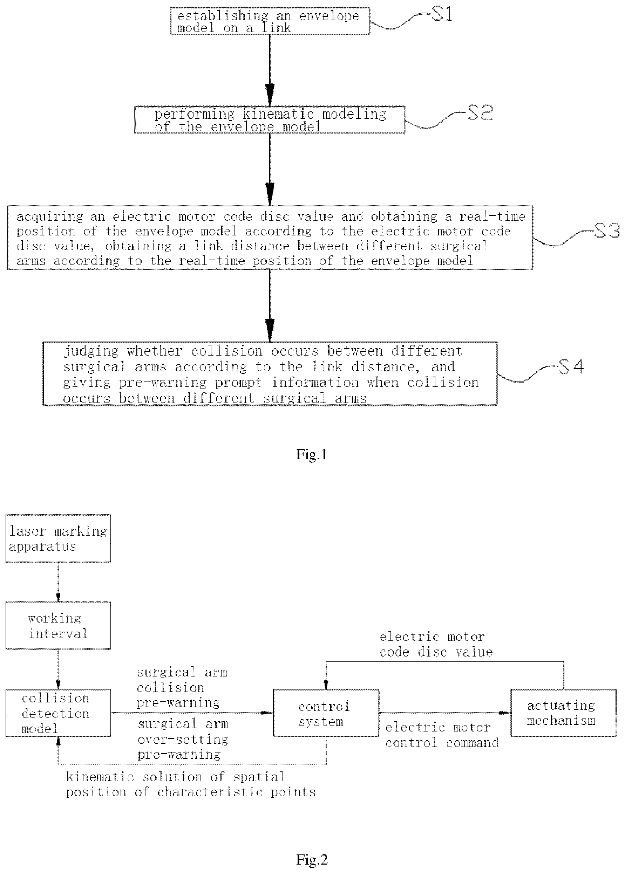

As shown in Fig. 1, an embodiment of the present disclosure provides a collision detection method of a laparoscopic minimally invasive surgery robot, comprising:

Step S1: an envelope model is established on the link, and thus the shape of the surgical arm in space can be seen as a series connection of several envelope models;

Step S2: kinematic modeling of envelope model is carried out;

Step S3: an electric motor code disc value is acquired and a real-time position of an envelope model is obtained according to the electric motor code disc value, and a link distance between different surgical arms (specifically, a mirror-holding arm and an instrument arm) is obtained according to the real-time position of the envelope model;

Step S4: whether collision occurs between different surgical arms is judged according to the link distance, and pre-warning prompt information is provided when collision occurs between different surgical arms.

In this embodiment, envelope models are created separately for potentially colliding links on different surgical arms, and are enabled to wrap the entire link or potentially colliding portions of the structure. Then, the control system performs kinematic modeling on the envelope model, as shown in Fig. 2, namely, completing the establishment of a collision detection model. The control system acquires the electric motor code disc values of the joint positions of the instrument arm and the mirror-holding arm and obtains the real-time spatial positions of each envelope model through calculation. According to the real-time spatial positions of the envelope models, the link distance, i.e. the distance between two different envelope models, can be easily calculated and obtained. Specifically, as shown in Fig. 2, the electric motor code disc values can be acquired by the actuating mechanism and sent to the control system. Finally, the control system determines whether there is a collision between two different surgical arms according to the link distance, and when there is a collision, the control system will send a pre-warning prompt information to notify the staff to adjust. At the same time, the control system also warns whether the surgical arm interferes with external equipment.

In summary, the real-time spatial position of the surgical arm can be obtained on the basis of the electric motor code disc value of the surgical arm, and the real-time spatial position of the surgical arm can be detected by using external sensors such as distance measurement, which reduces the dependence on detection apparatuses such as sensors, eliminates the integration problem during the use of multiple sensors and saves costs. At the same time, through the active detection of the link distance during the operation of the surgical arms, the collision between the surgical arms and the interference with the external equipment can be effectively warned, avoiding the intraoperative safety problems caused by the collision and facilitating the operation of laparoscopic minimally invasive surgery.

Optionally, a manner of establishing the envelope model comprises: determining a position of a characteristic point on a link, and establishing an envelope model by means of the characteristic point, a preset radius of the envelope model and a preset length of the envelope model, wherein the length of the envelope model is a distance length between two characteristic points.

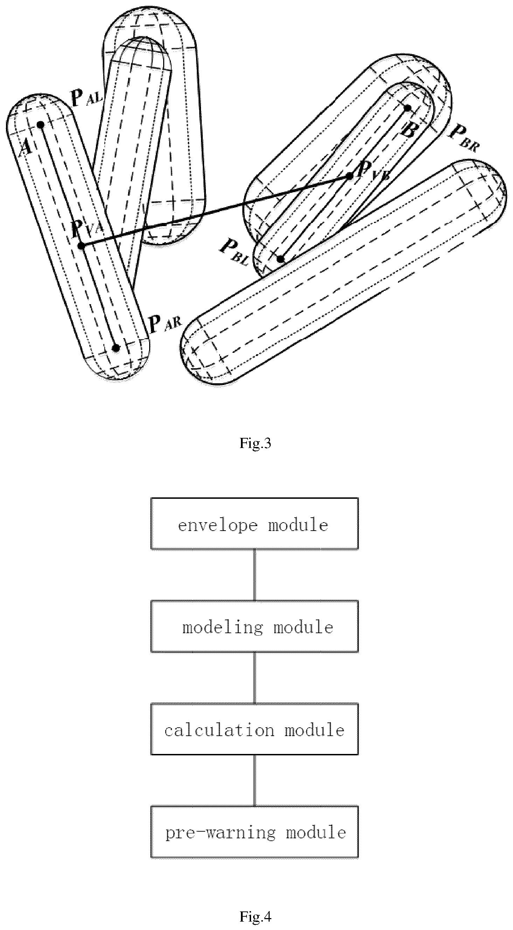

In this embodiment, since the link structure of the instrument arm and the mirror-holding arm is complicated, it is not easy to analyze the spatial position of the link of the instrument arm and the mirror-holding arm, and it is convenient to analyze by abstracting the link of the instrument arm and the mirror-holding arm as an envelope model. Specifically, through the evaluation of the link structure, the positions of two characteristic points on the link, the radius of the envelope model and the length of the envelope model are designed, and then the envelope model is established on the basis of the two characteristic points, the radius and the length. Specifically, as shown in Fig. 3, in the present embodiment, taking a link of a pair of surgical arms that may collide as an example, an envelope model A and an envelope model B are respectively established for the instrument arm and the mirror-holding arm link, wherein the characteristic points of the envelope model A are $P_{AL}$ and $P_{AR}$, and the axial segment $P_{AL}P_{AR}$ is a connection line of the characteristic points $P_{AL}$ and $P_{AR}$, and the characteristic points of the envelope model B are $P_{BL}$ and $P_{BR}$, and the axial segment $P_{BL}P_{BR}$ is a connection line of the characteristic points $P_{BL}$ and $P_{BR}$.

Optionally, as shown in Fig. 3, the shortest distance from any point on the axial segment of the envelope model to the housing of the envelope model, i.e. the outer surface of the envelope model, is equal.

In this embodiment, the envelope model is arranged as a combination of one cylinder and two hemispheres, wherein the radius of the cylinder and the radius of the hemisphere are the same, in particular a capsule-like shape. The characteristic points $P_{AL}$ and $P_{AR}$ are respectively the centres of the hemispheres on both ends of the envelope model A, and the characteristic points $P_{BL}$ and $P_{BR}$ are respectively the centres of the hemispheres on both ends of the envelope model B. Thus, it can be seen that the shortest distance from any point on the axial segment of the envelope model to the housing of the envelope model is the radius of the envelope model, thereby simplifying the structure of the link of the surgical arm, and further facilitating the calculation and analysis of the spatial position of the link of the surgical arm.

Optionally, as shown in Fig. 3, the way of obtaining the real-time position of the envelope model comprises: the real-time position of the characteristic point is obtained by performing kinematic solution according to the electric motor code disc value, and the real-time position of the envelope model is obtained by calculating according to the real-time position of the characteristic point and the radius of the envelope model.

In the present embodiment, the control system can obtain real-time spatial positions of two different characteristic points $P_{AL}$ and $P_{AR}$ of the envelope model A by means of a kinematic solution according to the electric motor code disc values, and likewise, the control system can obtain real-time spatial positions of two different characteristic points $P_{BL}$ and $P_{BR}$ of the envelope model B by means of a kinematic solution according to the electric motor code disc values. Since the characteristic points $P_{AL}$ and $P_{AR}$ are on the envelope model A, and the radius of the envelope model A is known at the same time, the spatial position of any point on the envelope model A can be obtained the real-time spatial position of the envelope model A. Similarly, since the characteristic points PBL and PBR are on the envelope model B, and the radius of the envelope model B is known at the same time, the spatial position of any point on the envelope model B can be obtained through calculation, thereby obtaining the real-time spatial position of the envelope model B.

Optionally, a manner of obtaining a link distance between different surgical arms comprises: calculating the shortest distance between the axial segment $P_{AL}P_{AR}$ and the axial segment $P_{BL}P_{BR}$ according to the real-time positions of the envelope model A and the envelope model B, and calculating the link distance according to the shortest distance.

In this embodiment, since it is complicated to calculate the link distance directly from the real-time positions of the envelope model A and the envelope model B, the shortest distance between the axial segment $P_{AL}P_{AR}$ and the axial segment $P_{BL}P_{BR}$ can be calculated first from the real-time positions of the envelope model A and the envelope model B, and then the link distance can be calculated and obtained more easily on the basis of the shortest distance.

Optionally, a manner of obtaining the shortest distance between the axial segment $P_{AL}P_{AR}$ and the axial segment $P_{BL}P_{BR}$ comprises:

determining a common perpendicular of an axis of the envelope model A and an axis of the envelope model B;

judging whether the intersection points of the common perpendicular line with the axis of the envelope model A and the axis of the envelope model B completely fall on the axial segment $P_{AL}P_{AR}$ and the axial segment $P_{BL}P_{BR}$;

when the intersection points of the common perpendicular line with the axis of the envelope model A and the axis of the envelope model B completely fall on the axial segment $P_{AL}P_{AR}$ and the axial segment $P_{BL}P_{BR}$, determining the shortest distance according to the following formula:

$$k_1 = \frac{(P_{BR} - P_{BL})^2 \cdot (P_{AR} - P_{AL}) \cdot (P_{AL} - P_{BL}) - (P_{AR} - P_{AL}) \cdot (P_{BR} - P_{BL})^2 \cdot (P_{AL} - P_{BL})}{(P_{AR} - P_{AL})^2 \cdot (P_{BR} - P_{BL})^2 - (P_{AR} - P_{AL})^2 \cdot (P_{BR} - P_{BL})^2}$$

$$k_2 = \frac{(P_{AR} - P_{AL})^2 \cdot (P_{BR} - P_{BL}) \cdot (P_{AL} - P_{BL}) - (P_{BR} - P_{BL}) \cdot (P_{BR} - P_{BL})^2 \cdot (P_{AL} - P_{BL})}{(P_{AR} - P_{AL})^2 \cdot (P_{BR} - P_{BL})^2 - (P_{AR} - P_{AL})^2 \cdot (P_{BR} - P_{BL})^2}$$

$$P_{VA} = P_{AL} + k_1(P_{AR} - P_{AL})$$

$$P_{VB} = P_{BL} + k_2(P_{BR} - P_{BL})$$

$$D_P = \| P_{VA} - P_{VB} \|$$

wherein $P_{VA}$ is the intersection point of the common perpendicular line with the axis of the envelope model A, $P_{VB}$ is the intersection point of the common perpendicular line with the axis of the envelope model B, and DP is the shortest distance between the axial segment $P_{AL}P_{AR}$ and the axial segment $P_{BL}P_{BR}$;

when the intersection points of the common perpendicular line with the axis of the envelope model A and the axis of the envelope model B fail to completely fall on the axial segment $P_{AL}P_{AR}$ and the axial segment $P_{BL}P_{BR}$, determining the shortest distance according to the following formula:

$$D_{AL}^B = \begin{cases} \| P_{AL} - P_{BL} \|, k < 0 \\ \| P_{AL} - kP_{BR} - (1-k)P_{BL} \|, 0 \le k \le 1, k = \dfrac{(P_{BR} - P_{BL}) \cdot (P_{AL} - P_{BL})}{(P_{BR} - P_{BL}) \cdot (P_{BR} - P_{BL})} \\ \| P_{AL} - P_{BR} \|, k > 1 \end{cases}$$

$$D_{AR}^B = \begin{cases} \| P_{AR} - P_{BL} \|, k < 0 \\ \| P_{AR} - kP_{BR} - (1-k)P_{BL} \|, 0 \le k \le 1, k = \dfrac{(P_{BR} - P_{BL}) \cdot (P_{AR} - P_{BL})}{(P_{BR} - P_{BL}) \cdot (P_{BR} - P_{BL})} \\ \| P_{AR} - P_{BR} \|, k > 1 \end{cases}$$

$$D_{BL}^{A} = \begin{cases} \|P_{BL} - P_{AL}\|, k < 0 \\ \|P_{BL} - kP_{AR} - (1-k)P_{AL}\|, 0 \le k \le 1, k = \dfrac{(P_{AR} - P_{AL}) \cdot (P_{BL} - P_{AL})}{(P_{AR} - P_{AL}) \cdot (P_{AR} - P_{AL})} \\ \|P_{BL} - P_{AR}\|, k > 1 \end{cases}$$

$$D_{BR}^{A} = \begin{cases} \|P_{BR} - P_{AL}\|, k < 0 \\ \|P_{BR} - kP_{AR} - (1-k)P_{AL}\|, 0 \le k \le 1, k = \dfrac{(P_{AL} - P_{BL}) \cdot (P_{BR} - P_{AL})}{(P_{AR} - P_{AL}) \cdot (P_{AR} - P_{AL})} \\ \|P_{BR} - P_{AR}\|, k > 1 \end{cases}$$

$$D_P = \min(D_{AR}^{B}, D_{AL}^{B}, D_{BL}^{A}, D_{BR}^{A})$$

wherein $D_{AL}^{B}$ and $D_{AR}^{B}$ are the shortest distances from two different characteristic points $P_{AL}$ and $P_{AR}$ to the axial segment $P_{BL}P_{BR}$, respectively, and $D_{BL}^{A}$ and $D_{BR}^{A}$ are the shortest distances from two different characteristic points $P_{BL}$ and $P_{BR}$ to the axial segment $P_{AL}P_{AR}$, respectively.

In the present embodiment, when the intersection point of the common perpendicular line with the axis of the envelope model A and the axis of the envelope model B completely falls on the axial segment $P_{AL}P_{AR}$ and the axial segment $P_{BL}P_{BR}$, according to the structural characteristics of the envelope model A and the envelope model B, it can be determined that the shortest distance between the axial segment $P_{AL}P_{AR}$ and the axial segment $P_{BL}P_{BR}$ is a minimum of the shortest distance from the characteristic points $P_{AL}$ and $P_{AR}$ to the axial segment $P_{BL}P_{BR}$ and the shortest distance from the characteristic points $P_{BL}$ and $P_{BR}$ to the axial segment $P_{AL}P_{AR}$, without calculating all the distances between the axial segment $P_{AL}P_{AR}$ and the axial segment $P_{BL}P_{BR}$, thereby simplifying the calculation process of the link distance. When the intersection point of the common perpendicular line with the axis of the envelope model A and the axis of the envelope model B completely falls on the axial segment $P_{AL}P_{AR}$ and the axial segment $P_{BL}P_{BR}$, according to the structural characteristics of the envelope model A and the envelope model B, it can be judged that the shortest distance between the axial segment $P_{AL}P_{AR}$ and the axial segment $P_{BL}P_{BR}$ is the distance between the two intersection points $P_{VA}$ and $P_{VB}$, without calculating all the distances between the axial segment $P_{AL}P_{AR}$ and the axial segment $P_{BL}P_{BR}$, thereby further simplifying the calculation process of the link distance.

Optionally, a manner of calculating the link distance according to the shortest distance between the axial segment $P_{AL}P_{AR}$ and the axial segment $P_{BL}P_{BR}$ comprises:

$$D_V = D_P - r_{SA} - r_{SB}$$

wherein $r_{SA}$ is the radius of envelope model A, $r_{SB}$ is the radius of envelope model B, and Dv is the link distance.

In the present embodiment, since the link distance is the shortest distance between the housing of the envelope model A and the housing of the envelope model B, and the shortest distance between the two different axial segments $P_{AL}P_{AR}$ and the axial segment $P_{BL}P_{BR}$ includes the shortest distance between the housing of the envelope model A and the housing of the envelope model B, the radius of the envelope model A, and the radius of the envelope model B, the link distance can be obtained by eliminating the radius of the envelope model A and the radius of the envelope model B through calculation.

Optionally, as shown in Fig. 2, the collision detection method of a laparoscopic minimally invasive surgery robot further comprises the following steps: defining the working interval of the surgical arm by a laser marking apparatus to define a range of motion of the surgical arm.

In this embodiment, the operating range of the instrument arm and the mirror-holding arm can be set by the laser marking apparatus of the robot, so that the instrument arm and the mirror-holding arm operate in a designated area, thereby avoiding interference from external devices and the environment. When the instrument arm or the mirror-holding arm exceeds the working interval defined by the laser marking apparatus, the collision detection model will send a pre-warning signal to the control system, and the control system will send a corresponding electric motor control command to the actuating mechanism according to the pre-warning signal, so as to control the instrument arm and the mirror-holding arm to stop so as to facilitate the adjustment by the operator.

Optionally, as shown in Figs. 1 and 2, a judgment manner of judging whether collision occurs between different surgical arms according to the link distance includes: comparing the link distance with a preset threshold value, and when the link distance is less than or equal to the threshold value, judging that collision occurs between different surgical arms, while when the link distance is greater than the threshold value, judging that no collision occurs between different surgical arms.

In the present embodiment, when the link distance is greater than a threshold value (the threshold value is not fixed and the same, and each pair of links that may collide has a corresponding threshold value), it means that the instrument arm and the lens-holding arm will not collide at this time, and thus the distance between the instrument arm and the lens-holding arm does not need to be adjusted during the surgery, and thus the pre-warning signal does not need to be generated. However, when the link distance is less than or equal to the threshold value, it indicates that the instrument arm and the mirror-holding arm will collide, and at this time, the collision detection model will send a pre-warning signal to the control system, and the control system will send a corresponding electric motor control command to the actuating mechanism according to the pre-warning signal, so as to control the instrument arm and the mirror-holding arm to stop the surgical operation, thereby facilitating human intervention by the operator.

As shown in Fig. 4, another embodiment of the present disclosure discloses a collision detection apparatus based on a laparoscopic minimally invasive surgery robot, comprising:

an envelope module for establishing an envelope model on a link;
a modeling module for performing kinematic modeling of the envelope model;
a calculation module for acquiring an electric motor code disc value and obtaining a real-time position of the envelope model according to the electric motor code disc value, and for obtaining a link distance between different surgical arms according to the real-time position of the envelope model;
an pre-warning module for judging whether collision occurs between different surgical arms according to the link distance, and providing pre-warning prompt information when collision occurs between different surgical arms.

In this embodiment, the envelope module creates an envelope model for each link of each surgical arm (instrument arm and mirror-holding arm), respectively, and enables the envelope model to wrap the entire link or the portion of the structure that may collide. Then, the modeling module performs kinematic modeling on the envelope model, namely, establishing a collision detection model. The calculation module obtains the electric motor code disc values of the joint positions of the instrument arm and the mirror-holding arm and obtains the real-time spatial position of the envelope model through calculation, and then can easily calculate and obtain the link distance, namely, the shortest distance of two different envelope models, according to the real-time spatial position of the envelope model. Finally, the pre-warning module judges whether there is a collision between two different surgical arms according to the link distance, and when collision occurs, the control system will send a pre-warning prompt information to notify the staff to adjust. At the same time, the pre-warning module also sends out pre-warning on whether the surgical arm interferes with external equipment.

Although the present disclosure has been described above, the scope of protection of the present disclosure is not limited thereto. Various changes and modifications may be effected by one skilled in the art without departing from the spirit and scope of the disclosure, and it is intended that such changes and modifications fall within the scope of protection of the present disclosure.

## Claims

1. A collision detection method of a laparoscopic minimally invasive surgery robot, comprising:

establishing an envelope model on a link,
performing kinematic modeling of the envelope model,
acquiring an electric motor code disc value and obtaining a real-time position of the envelope model according to the electric motor code disc value, obtaining a link distance between different surgical arms according to the real-time position of the envelope model,
judging whether collision occurs between different surgical arms according to the link distance, and
providing pre-warning prompt information when collision occurs between different surgical arms.

2. The collision detection method of a laparoscopic minimally invasive surgery robot according to claim 1, wherein a manner of establishing the envelope model comprises:
determining a position of a characteristic point on the link, and establishing the envelope model according to the characteristic point, a preset radius of the envelope model and a preset length of the envelope model.

3. The collision detection method of a laparoscopic minimally invasive surgery robot according to claim 2, wherein a shortest distance from any point on an axial segment of the envelope model to a housing of the envelope model is equal.

4. The collision detection method of a laparoscopic minimally invasive surgery robot according to claim 3, wherein a manner of obtaining a real-time position of the envelope model comprises: obtaining the real-time position of the characteristic point by performing a kinematic solution according to the electric motor code disc value, and obtaining the real-time position of the envelope model by calculating according to the real-time position of the characteristic point and the radius of the envelope model.

5. The collision detection method of a laparoscopic minimally invasive surgery robot according to claim 4, wherein a manner of obtaining a link distance between different surgical arms comprises: obtaining a shortest distance between two different axial segments according to a real-time position of the envelope model, and obtaining the link distance according to the shortest distance.

6. The collision detection method of a laparoscopic minimally invasive surgery robot according to claim 5, wherein a manner of obtaining a shortest distance between two different axial segments comprises:

determining a common perpendicular line of axes of two different envelope models,
judging whether intersection points of the common perpendicular line with axes of two different envelope models completely fall on two different axial segments,
determining the shortest distance according to the following formula when the intersection points of the common perpendicular line with the axes of two different envelope models completely fall on two different axial segments:

$$k_1 = \frac{(P_{BR} - P_{BL})^2 \cdot (P_{AR} - P_{AL}) \cdot (P_{AL} - P_{BL}) - (P_{AR} - P_{AL}) \cdot (P_{BR} - P_{BL})^2 \cdot (P_{AL} - P_{BL})}{(P_{AR} - P_{AL})^2 \cdot (P_{BR} - P_{BL})^2 - (P_{AR} - P_{AL})^2 \cdot (P_{BR} - P_{BL})^2}$$

$$k_2 = \frac{(P_{AR} - P_{AL})^2 \cdot (P_{BR} - P_{BL}) \cdot (P_{AL} - P_{BL}) - (P_{BR} - P_{BL}) \cdot (P_{BR} - P_{BL})^2 \cdot (P_{AL} - P_{BL})}{(P_{AR} - P_{AL})^2 \cdot (P_{BR} - P_{BL})^2 - (P_{AR} - P_{AL})^2 \cdot (P_{BR} - P_{BL})^2}$$

$$P_{VA} = P_{AL} + k_1 (P_{AR} - P_{AL})$$

$$P_{VB} = P_{BL} + k_2 (P_{BR} - P_{BL})$$

$$D_P = \| P_{VA} - P_{VB} \|$$

wherein $P_{AL}$ and $P_{AR}$ are respectively two different characteristic points of one envelope model, while $P_{BL}$ and $P_{BR}$ are respectively two different characteristic points of the other envelope model, $P_{VA}$ being the intersection point of the common perpendicular line with the axis of one envelope model, $P_{VB}$ being the intersection point of the common perpendicular line with the axis of the other envelope model, and $D_P$ being the shortest distance, determining the shortest distance according to the following formula when the intersection point of the common perpendicular line with the axes of two different envelope models fail to completely fall on two different axial segments:

$$D_{AL}^B = \begin{cases} \| P_{AL} - P_{BL} \|, k < 0 \\ \| P_{AL} - k P_{BR} - (1-k) P_{BL} \|, 0 \le k \le 1, k = \frac{(P_{BR} - P_{BL}) \cdot (P_{AL} - P_{BL})}{(P_{BR} - P_{BL}) \cdot (P_{BR} - P_{BL})} \\ \| P_{AL} - P_{BR} \|, k > 1 \end{cases}$$

$$D_{AR}^{B} = \begin{cases} \left\| P_{AR} - P_{BL} \right\|, k < 0 \\ \left\| P_{AR} - kP_{BR} - (1-k)P_{BL} \right\|, 0 \le k \le 1, k = \dfrac{(P_{BR} - P_{BL}) \cdot (P_{AR} - P_{BL})}{(P_{BR} - P_{BL}) \cdot (P_{BR} - P_{BL})} \\ \left\| P_{AR} - P_{BR} \right\|, k > 1 \end{cases}$$

$$D_{BL}^{A} = \begin{cases} \left\| P_{BL} - P_{AL} \right\|, k < 0 \\ \left\| P_{BL} - kP_{AR} - (1-k)P_{AL} \right\|, 0 \le k \le 1, k = \dfrac{(P_{AR} - P_{AL}) \cdot (P_{BL} - P_{AL})}{(P_{AR} - P_{AL}) \cdot (P_{AR} - P_{AL})} \\ \left\| P_{BL} - P_{AR} \right\|, k > 1 \end{cases}$$

$$D_{BR}^{A} = \begin{cases} \left\| P_{BR} - P_{AL} \right\|, k < 0 \\ \left\| P_{BR} - kP_{AR} - (1-k)P_{AL} \right\|, 0 \le k \le 1, k = \dfrac{(P_{AL} - P_{BL}) \cdot (P_{BR} - P_{AL})}{(P_{AR} - P_{AL}) \cdot (P_{AR} - P_{AL})} \\ \left\| P_{BR} - P_{AR} \right\|, k > 1 \end{cases}$$

$$D_{P} = \min(D_{AR}^{B}, D_{AL}^{B}, D_{BL}^{A}, D_{BR}^{A})$$

wherein $D_{AL}^{B}$ and $D_{AR}^{B}$ are respectively the shortest distances of two different characteristic points $P_{AL}$ and $P_{AR}$ to one axial segment, and $D_{BL}^{A}$ and $D_{BR}^{A}$ are respectively the shortest distances of two different characteristic points $P_{BL}$ and $P_{BR}$ to the other axial segment.

7. The collision detection method of a laparoscopic minimally invasive surgery robot according to claim 6, wherein a manner of obtaining the link distance according to the shortest distance comprises:

$$D_{V} = D_{P} - r_{SA} - r_{SB}$$

wherein $r_{SA}$ is a radius of one envelope model, $r_{SB}$ is a radius of the other envelope model, and Dv is the link distance.

8. The collision detection method of a laparoscopic minimally invasive surgery robot according to any one of claims 1-7, further comprising defining a working interval of the surgical arm with a laser marking apparatus to define a range of motion of the surgical arm.

9. The collision detection method of a laparoscopic minimally invasive surgery robot according to claim 1, wherein a manner of judging whether collision occurs between different surgical arms according to the link distance comprises: comparing the link distance with a preset threshold value, and when the link distance is less than or equal to the threshold value, judging that collision occurs between different surgical arms, while when the link distance is greater than the threshold value, judging that no collision occurs between different surgical arms.

10. A collision detection apparatus based on a laparoscopic minimally invasive surgery robot, comprising:

an envelope module for establishing an envelope model on a link,
a modeling module for performing kinematic modeling of the envelope model so as to calculate a real-time spatial position of the envelope model,
a calculation module for acquiring an electric motor code disc value and obtaining a real-time position of the envelope model according to the electric motor code disc value and the established kinematic model, and for obtaining a link distance between different surgical arms according to the real-time position of the envelope model,
a pre-warning module for judging whether collision occurs between different surgical arms according to the link distance, and providing pre-warning prompt information when collision occurs between different surgical arms.

establishing an envelope model on a link ~S1

performing kinematic modeling of the envelope model ~S2

acquiring an electric motor code disc value and obtaining a real-time position of the envelope model according to the electric motor code disc value, obtaining a link distance between different surgical arms according to the real-time position of the envelope model ~S3

judging whether collision occurs between different surgical arms according to the link distance, and giving pre-warning prompt information when collision occurs between different surgical arms ~S4

Fig.1

laser marking apparatus

working interval

collision detection model

surgical arm collision pre-warning

surgical arm over-setting pre-warning

kinematic solution of spatial position of characteristic points

control system

electric motor code disc value

electric motor control command

actuating mechanism

Fig.2

Fig.3

Fig.4

### INTERNATIONAL SEARCH REPORT

| International application No. |
|---|
| **PCT/CN2021/093793** |

| A. | CLASSIFICATION OF SUBJECT MATTER |
|---|---|
| | A61B 34/30(2016.01)i |

According to International Patent Classification (IPC) or to both national classification and IPC

| B. | FIELDS SEARCHED |
|---|---|

Minimum documentation searched (classification system followed by classification symbols)

A61B; B25J

Documentation searched other than minimum documentation to the extent that such documents are included in the fields searched

Electronic data base consulted during the international search (name of data base and, where practicable, search terms used)

WPI, EPODOC, CNPAT, CNKI: 思哲睿, 王伟, 文洋, 碰, 撞, 障, 避, 防, 检测, 包络, 包围, 间距, 距离, 机器人, 机械臂, 模型, 位置, 运动, collision, robot+, model, envelope, position, location, distance, space, collid???, arm

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 112773506 A (HARBIN SIZHERUI INTELLIGENT MEDICAL EQUIPMENT CO., LTD.) 11 May 2021 (2021-05-11) claims 1-10, description paragraphs [0004]-[0093], figures 1-4 | 1-10 |
| X | CN 111546376 A (HARBIN INSTITUTE OF TECHNOLOGY) 18 August 2020 (2020-08-18) description, paragraphs [0028]-[0065], and figures 1-4 | 1-5, 8-10 |
| A | CN 109015644 A (XU, Runqiu) 18 December 2018 (2018-12-18) entire document | 1-10 |
| A | CN 105479490 A (HUAZHONG UNIVERSITY OF SCIENCE AND TECHNOLOGY) 13 April 2016 (2016-04-13) entire document | 1-10 |
| A | CN 111203916 A (SHANXI WANHE INTELLIGENT TECHNOLOGY CO., LTD.) 29 May 2020 (2020-05-29) entire document | 1-10 |
| A | CN 110421556 A (HEBEI UNIVERSITY OF TECHNOLOGY) 08 November 2019 (2019-11-08) entire document | 1-10 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 September 2021** | **30 September 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2021/093793**

**C.      DOCUMENTS CONSIDERED TO BE RELEVANT**

| Category* | Citation of document, with indication, where appropriate, of the relevant passages | Relevant to claim No. |
|---|---|---|
| A | CN 110053043 A (SOUTH CHINA UNIVERSITY OF TECHNOLOGY) 26 July 2019 (2019-07-26)<br>      entire document | 1-10 |
| A | CN 111546378 A (HARBIN INSTITUTE OF TECHNOLOGY) 18 August 2020 (2020-08-18)<br>      entire document | 1-10 |
| A | CN 109634284 A (ANHUI POLYTECHNIC UNIVERSITY) 16 April 2019 (2019-04-16)<br>      entire document | 1-10 |
| A | CN 112264989 A (HUAZHONG UNIVERSITY OF SCIENCE AND TECHNOLOGY) 26 January 2021 (2021-01-26)<br>      entire document | 1-10 |
| A | WO 2020190832 A1 (COVIDIEN LP.) 24 September 2020 (2020-09-24)<br>      entire document | 1-10 |

Form PCT/ISA/210 (second sheet) (January 2015)

**INTERNATIONAL SEARCH REPORT**
Information on patent family members

International application No.

**PCT/CN2021/093793**

| Patent document cited in search report | | | Publication date (day/month/year) | Patent family member(s) | Publication date (day/month/year) |
|---|---|---|---|---|---|
| CN | 112773506 | A | 11 May 2021 | None | |
| CN | 111546376 | A | 18 August 2020 | None | |
| CN | 109015644 | A | 18 December 2018 | None | |
| CN | 105479490 | A | 13 April 2016 | None | |
| CN | 111203916 | A | 29 May 2020 | None | |
| CN | 110421556 | A | 08 November 2019 | None | |
| CN | 110053043 | A | 26 July 2019 | None | |
| CN | 111546378 | A | 18 August 2020 | None | |
| CN | 109634284 | A | 16 April 2019 | None | |
| CN | 112264989 | A | 26 January 2021 | None | |
| WO | 2020190832 | A1 | 24 September 2020 | None | |

Form PCT/ISA/210 (patent family annex) (January 2015)